# EUROPEAN PATENT SPECIFICATION

(11) **EP 4 277 678 B1**
(45) Date of publication and mention of the grant of the patent: **20.05.2026**
(21) Application number: 22700646.7
(22) Date of filing: 14.01.2022
(51) Int. Cl.: A61M 5/20, A61M 5/315, A61M 5/24

(54) **A TORSION SPRING DRIVEN FIXED DOSE INJECTION DEVICE**
INJEKTIONSVORRICHTUNG MIT TORSIONSFEDERANTRIEB
DISPOSITIF D'INJECTION ENTRAÎNÉ PAR UN RESSORT DE TORSION

(30) Priority: 18.01.2021 EP 21152143
(43) Date of publication of application: 22.11.2023
(73) Proprietor: Novo Nordisk A/S, 2880 Bagsværd (DK)
(72) Inventor: JAKOBSEN, Nikolaj, Eusebius, 2880 Bagsværd (DK)
(86) International application number: PCT/EP2022/050769
(87) International publication number: WO 2022/152864

(56) References cited:
- WO-A1-2020/089167
- DE-A1- 102006 038 102
- US-A1- 2013 096 513
- US-A1- 2015 165 130
- US-A1- 2017 136 186

## Description

### THE TECHNICAL FIELD OF THE INVENTION:

The invention relates to a torsion spring driven injection device for expelling a number of substantially equally sized dose volumes of a liquid drug and in particular to a torsion spring driven injection device wherein the doses to be expelled are predetermined by the manufacturer of the injection device and fixed in volume such that each individual dose has substantially the same volume.

### DESCRIPTION OF RELATED ART:

Spring driven injection devices for the automatic injection of dose volumes of a liquid drug are widely known in the art. Some of these injection devices uses a compression spring for driving out the dose volume and other uses a torsion spring.

When injecting a liquid solution containing insulin it is usually considered important that the user has a large range of different dose volumes available to choose between in order to inject exactly the necessary dose volume for the individual user for each injection. Such injection device as e.g. exemplified in US 6,899,699 are consequently provided with a dose setting mechanism by which the user incrementally can select the individual size of the dose volume to be injected. The dose expelling mechanism in US 6,899,699 is automatic and driven by a spring which can either be a compression spring or a torsion spring.

However, for other types of liquid drugs such as GLP-1 or the like, the need is to inject one and the same dose volume in each and every injection. Such injection devices are often referred to a fixed dose devices since the dose volume is fixed by the manufacturer of the injection device.

In one aspect this can be executed by having a single use injection device which is able to expel one single dose volume having a predetermined and fixed size. The user thus performs the injection and discards the single use injection device once it has been used.

However, an alternative to using a plurality of such single use fixed dose devices is to use one injection device which are dedicated for expelling a plurality of predetermined and equally sized dose volumes. Such injection devices are sometimes referred to as multi-use fixed dose injection devices.

Examples of such multi-use fixed dose device for expelling a plurality of predetermined and equally sized dose volumes are disclosed in WO 2017/098460, WO 2018/007259 and in WO 2020/089167.

Common for these injection devices are that the user prior to performing each and every injection must rotate a dose selection button which selects the predetermined sized dose volume, and which also strains a spring such that a force is build up and stored in the spring. The user thus needs rotatably to arm the injection device prior to performing each injection as the spring is manually strained to perform one single injection at the time.

In WO 2020/089167, the user strains a torsion spring by rotating the dose selection button and pushes an injection button provided proximally on the injection device to release the stored torque of the torsion spring which torque hereafter drives the piston rod in the distal direction such that the predetermined sized dose volume is pressed out from the injection device.

However, the multi-use fixed dose injection device disclosed in WO 2020/089167 comprises a high number of complicated parts.

### DESCRIPTION OF THE INVENTION:

It is henceforth an object of the present invention to provide a multi-use fixed dose injection device having a more simplified construction.

The invention is defined in claim 1. Advantageous embodiments are further defined in the dependent claims.

Accordingly, in one aspect of the present invention, a torsion spring driven injection device for ejecting a number of predetermined and substantially equally sized fixed dose volumes of a liquid drug comprises:
A housing structure which secures a cartridge containing the liquid drug to be dispensed,
A piston rod for driving the liquid drug out from the cartridge preferably by moving the piston rod in the distal direction,
A rotatable drive structure engaging the piston rod such that the piston rod is rotated together with the rotatable drive structure during dosing,
A nut element secured to the housing structure at least during dosing and threaded to the piston rod such that piston rod is moved helically upon rotation of the piston rod relatively to the nut element,
A connector element which is coupled to the housing structure by a ratchet interface allowing the connector element to rotate relatively to the housing structure,
A torsion spring which is operable coupled between the rotatable drive structure and the connector element, such that a torque is build up in the torsion spring by rotation of the connector element relative to the rotatable drive structure and wherein the ratchet interface between the housing structure and the connector element is able to hold the connector element in its rotational position against the torque of the torsion spring,
The rotatable drive structure being axially movable between a first position and a second position, wherein
   the first position is a locked position wherein the rotatable drive structure is non-rotationally secured to the housing structure, and
   the second position is a released position wherein the rotatable drive structure is released from the housing structure and able to rotate under influence of the torque stored in the torsion spring.
The torsion spring is strained when selecting one of the numbers of predetermined and substantially equally sized fixed dose volumes_by rotation of the connector element relatively to the rotatable drive structure with the rotatable drive structure maintained in the first position; the connector element being rotatable from an initial position to a dose position, and
wherein the selected one of the numbers of predetermined and substantially equally sized fixed dose volumes is individually released when the rotatable drive structure is moved axially from the first position and into the second position allowing the rotatable drive structure to rotate from the dose position to the initial position under influence of the torsion spring, and wherein,
the rotatable drive structure and the connector element are provided with means allowing the rotatable drive structure to move axially from the first position to the second position when the rotatable drive structure has been rotated to, and is positioned, in the dose position.

The fixed dose to be ejected is thus selected by rotating the connector element relatively to the rotational drive structure whereby the torsion spring is strained. The rotational drive structure and the connector element are for this purpose provided with means allowing the rotational drive structure to move from the first position and into the second position when these means are aligned. Such means could in one example be tracks and protrusions provided on either of the parts or the means could be solely protrusions provided on both parts. Other kind of mechanical means could be envisaged as long as the rotational drive structure is only allowed to be moved axially in the correct rotational position i.e. in the defined dose position. Hence during the rotation, it is not possible to move the rotational drive structure into the second released position. Only when the full fixed dose has been selected and the connector element has been rotated to the specific dose position is it possible to move the rotational drive structure from the first locked position to the second released position and hence release the fixed dose.

Hence two relative rotational positions (an initial position and a dose position) of the connector element and the drive structure are specified, and the size of the dose is predetermined by the rotational distance between these two positions; the initial position and the dose position thus defines the size of the fixed dose.

During dose setting the user hence rotates the connector element from the initial position to a dose position while the rotational drive structure is locked to the housing. This rotation strains the torsion spring. During dose release, the connector element is locked to the housing and the rotatable drive structure is rotated from the dose position and back to the initial position by the torsion spring.

It is understood that a fixed dose volume herein means a fixed metric volume of the liquid drug. Certain tolerances however apply such that a small variation in the volumes of each of the fixed doses are acceptable under the terms predetermined fixed dose volumes.

In order to provide a better grip for the user, a rotational selection element for rotational selecting the fixed dose to be ejected is provided, preferably at a proximal end of the housing structure. In one example, the rotational selection element is rotationally locked to the connector element such that the connector element rotates together one-to-one with the selection element when the user selects the fixed dose by rotation of the rotational selection element. The selection element is further axially movable relatively to the connector element, such that the selection element can be moved axially in relation to the connector element.

The rotational selection element can further be used as a dose release element by being axially coupled to the rotational drive structure such that the selection element and the rotational drive structure move together in the axial direction to move the rotational drive structure from the first position to the second position to thereby release the rotational drive structure and wherein the selection element and the rotational drive structure are allowed to rotate in relation to each other. It is thus possible to move the rotational drive structure from the locked position to the released position by axially pushing on the rotational selection element. The connection between the rotational selection element and the rotational drive structure can be any kind of coupling allowing relative rotation between the two parts but locking the two parts to move axially in unison. This could e.g. be a ridge or the like engaging or gripping behind a flange and could in one example include a needle or ball bearing or other means to lower friction during relative rotation between the two parts.

In order to move the rotational drive structure back to the first locked position a compression spring is preferably provided which urges the rotational selection element and the rotational drive structure in the proximal direction. Further, guiding means for guiding the selection element is preferably provided in the housing structure. Such guiding means are preferable splines being guided in one or more tracks.

In one specific example the rotational drive structure comprises a longitudinal drive tube provided with a radial protrusion as one of the means allowing the drive tube to move axially from the first position to the second position. By tube is here meant a longitudinal shaped element which preferably, but not necessarily, is hollow or at least partly hollow. The radial protrusion is preferably located on the outer surface and preferably in the proximity of the proximal end of the drive tube.

Preferably, the drive tube extends through the connector element such that the radial protrusion can engage the connector element.

The connector element is in a further example provided with a first axial opening, as one of the means allowing the drive tube to move axially from the first position to the second position, through which opening the radial protrusion provided on the drive tube is able to move when the first axial opening and the radial protrusion are aligned (the dose position) to thereby initiate an ejection. It is thus possible to move the drive tube from the first locked position to the second released position in this specific dose position.

The connector element is preferably also provided with a second axial opening such that the radial protrusion provided on the drive tube is able to pass axially through the second axial opening in the connector element when the second axial opening and the radial protrusion are aligned (the initial position) at the end of an ejection. The drive tube is thus movable from the second released position to the first locked position in this initial position following an ejection of the fixed dose.

The piston rod used for expelling the fixed dose is preferably provided with a helical outer thread and a longitudinal guiding structure such as a groove or a flat surface. In such construction the piston rod can be rotated by having an element such as the drive structure engage the longitudinal guiding structure in a key-like engagement such that the piston rod rotates together with the drive structure.

When the piston rod is rotated it is moved in the distal direction by an engagement with a nut element carried by the housing structure. The nut element has an inner thread engaging the outer thread on the piston rod and whenever the drive tube, provided with engaging means engaging the longitudinal guiding structure on the piston rod, is rotated, the piston rod is screwed forward in the distal direction in a helical movement.

In one example, the nut element is axially movable and coupled to the drive tube to move axially together with the drive tube. It is thus possible to obtain a so-called floating nut which can be coupled to and de-coupled from the housing. The principle of such floating nut is further described in detail in EP 2,906,271. However, the nut element could also be permanently connected to the housing.

By coupling the nut element to the drive tube, it is possible to rotational release the nut element from the housing structure in the first position of the drive tube and rotationally secure to the nut element to the housing structure in the second position of the drive tube. Hence, the nut element is only coupled to the housing during dose expelling but is free to rotate during dose selection and during change of the cartridge.

### DEFINITIONS:

An **"injection pen"** is typically an injection apparatus having an oblong or elongated shape somewhat like a pen for writing. Although such pens usually have a tubular cross-section, they could easily have a different cross-section such as triangular, rectangular or square or any variation around these or other geometries.

The term **"Needle Cannula"** is used to describe the actual conduit performing the penetration of the skin during injection. A needle cannula is usually made from a metallic material such as e.g. stainless steel and preferably connected to a hub made from a suitable material e.g. a polymer. A needle cannula could however also be made from a polymeric material or a glass material. The needle cannula mounted in the hub and referred to as the injection needle or the needle assembly can either be exchangeable or permanently attached to the injection device. A special needle assembly is the so-called **"Pen Needle"** wherein a part of the needle cannula extends in the proximal direction from the base of the hub such that this proximal part can penetrate into the cartridge once the pen needle is attached to the injection device.

As used herein, the term **"Liquid drug"** is meant to encompass any drug-containing flowable medicine capable of being passed through a delivery means such as a hollow needle cannula in a controlled manner, such as a liquid, solution, gel or fine suspension. Representative drugs could include pharmaceuticals such as peptides, proteins (e.g. insulin, insulin analogues and C-peptide), and hormones, biologically derived or active agents, hormonal and gene based agents, nutritional formulas and other substances in both solid (dispensed) or liquid form.

**"Cartridge"** is the term used to describe the primary container actually containing the liquid drug. Cartridges are usually made from glass e.g. borosilicate glass but could alternatively be moulded from any suitable polymer. A cartridge or ampoule is preferably sealed at one end by a pierceable membrane referred to as the **"septum"** which can be pierced e.g. by the non-patient end of a needle cannula. Such septum is usually self-sealing which means that the opening created during penetration seals automatically by the inherent resiliency once the needle cannula is removed from the septum. The opposite end of the cartridge is typically closed by a plunger or piston made from a rubber composition or a suitable polymer. The plunger or piston can be slidable moved inside the cartridge. The space between the pierceable membrane and the movable plunger holds the liquid drug which is pressed out as the plunger decreased the volume of the space holding the liquid drug.

Since a cartridge usually has a narrow distal neck portion into which the plunger cannot be moved not all of the liquid drug contained inside the cartridge can actually be expelled. The term **"initial quantum"** or **"substantially used"** therefore refers to the injectable content contained in the cartridge and thus not necessarily to the entire content. The injectable content in the cartridge must be at least equal to the volume making up the plurality of the predetermined sized dose volumes to be expelled. If in one example the multi-use fixed dose injection device is supposed to contain three fixed doses each having a volume of e.g. 0,3 ml, the injectable content of the cartridge needs to be at least 0,9 ml and the full volume of the cartridge must thus be larger to also include the volume that cannot be expelled due to the narrow neck part.

By the term **"Pre-filled"** injection device is meant an injection device in which the cartridge containing the liquid drug is permanently embedded in the injection device such that it cannot be removed without permanent destruction of the injection device. Once the predetermined amount of liquid drug in the cartridge is used, the user normally discards the entire injection device. Usually, the cartridge which has been filled by the manufacturer with a specific amount of liquid drug is secured in a cartridge holder which is then permanently connected in a housing structure such that the cartridge cannot be exchanged.

This is in opposition to a **"Durable"** injection device in which the user can himself change the cartridge containing the liquid drug whenever it is empty. Pre-filled injection devices are usually sold in packages containing more than one injection device whereas durable injection devices are usually sold one at a time. When using pre-filled injection devices an average user might require as many as 50 to 100 injection devices per year whereas when using durable injection devices one single injection device could last for several years, however, the average user would require 50 to 100 new cartridges per year.

A **"Multi-Use Fixed Dose"** injection device is meant to define an injection device which is able to deliver a predefined plurality (i.e. more than one) of doses which are substantially identical in volume. The initial quantum of liquid drug contained in the cartridge is thus expelled in a number of substantially identical dose volumes. In one example the cartridge could e.g. have an initial injectable quantum of 3 ml of liquid drug which could e.g. be expelled in 6 identical doses each of 0,5 ml. The number of equally sized dose volumes are often 2 to 8, and preferably 4 to 6 identical dose volumes. A multi-use fixed dose injection device can either be pre-filled such that the entire injection device is discarded after the predefined number of dose volumes has been expelled or it can be a durable injection device enabling the user to change the cartridge and expel a new series of equally sized doses volumes from the new cartridge.

Using the term **"Automatic"** in conjunction with injection device means that, the injection device is able to perform the injection without the user of the injection device delivering the force needed to expel the liquid drug during dosing. The force is typically delivered - automatically - by an electric motor or by a spring drive. The actual spring for the spring drive is e.g. strained by the user during dose setting, however, such springs are usually pre-strained with a low force in order to avoid problems of delivering very small doses. Alternatively, the spring can be fully preloaded by the manufacturer with a preloaded force sufficient to expel the full initial content (i.e. the entire injectable content) of liquid drug contained in the cartridge though a number of doses. Typically, the user activates a release mechanism provided either on the surface of the housing or at the proximal end of the injection device to partially release some of the force accumulated in the spring when carrying out the injection. Alternatively, the injection device can be shield triggered such that the activation of a movable shield releases the force required to expel the dose.

The term **"Permanently connected"** or **"permanently embedded"** as used in this description is intended to mean that the parts, and especially the cartridge is permanently embedded in the housing structure and requires the use of tools in order to be separated and should the parts be separated it would permanently damage at least one of the parts thus rendering the injection device unable to operate.

All headings and sub-headings are used herein for convenience only and should not be constructed as limiting the invention in any way.

The use of any and all examples, or exemplary language (e.g. such as) provided herein, is intended merely to better illuminate the invention and does not pose a limitation on the scope of the invention unless otherwise claimed. No language in the specification should be construed as indicating any non-claimed element as essential to the practice of the invention.

The citation and incorporation of patent documents herein is done for convenience only and does not reflect any view of the validity, patentability, and/or enforceability of such patent documents.

The invention is defined by the appended claims.

### BRIEF DESCRIPTION OF THE DRAWINGS:

The invention will be explained more fully below in connection with a preferred embodiment and with reference to the drawings in which:
- Figure 1: show an exploded view of the injection device according to the disclosed example of the invention.
- Figure 2A-B: show cross-sectional views of the injection device disclosed in figure 1. Figure 2B being an enlargement of the encircled part of figure 2A.
- Figure 3A-B: show two different perspective views of the connector element. Figure 3A being viewed from a proximal position.
- Figure 4: show a perspective view of the drive tube.
- Figure 5A-B: show two different views of the selector element. Figure 5A being a cross-sectional view.
- Figure 6A-B: show two different perspective views of the interface between the housing part and the connector element.
- Figure 7: show a cross-sectional view of the drive mechanism.

The figures are schematic and simplified for clarity, and they just show details, which are essential to the understanding of the invention, while other details are left out. Throughout, the same reference numerals are used for identical or corresponding parts.

### DETAILED DESCRIPTION OF EMBODIMENT:

When in the following terms as "upper" and "lower", "right" and "left", "horizontal" and "vertical", "clockwise" and "counter-clockwise" or similar relative expressions are used, these only refer to the appended figures and not necessarily to an actual situation of use. The shown figures are schematic representations for which reason the configuration of the different structures as well as their relative dimensions are intended to serve illustrative purposes only.

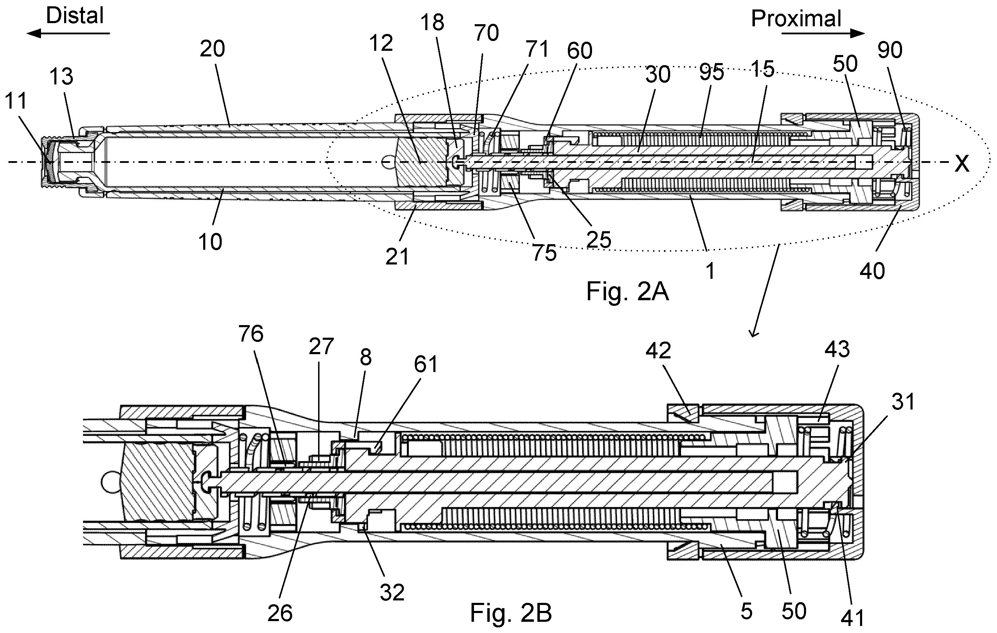

In that context it may be convenient to define that the term "distal end" in the appended figures is meant to refer to the end of the injection device usually carrying the needle cannula, whereas the term "proximal end" is meant to refer to the opposite end usually carrying the dose selection button as depicted in figure 2A. Distal and proximal is meant to be along an axial orientation extending along the longitudinal axis (X) of the medical container as also shown in figure 2A.

When referring to clock-wise and anti or counter clock-wise in the following examples it is understood that the injection device is viewed from a position distal to the injection device. Clock-wise is thus a rotation following the arms on an ordinary clock and counter clock-wise is a rotation in the opposite direction.

To explain the various movements taken place in the injection device described in the example, the following terminology are used throughout the following detailed description;

"Translational movement" is meant to be a strictly linear movement without any rotation.

"Rotational movement" is any movement of rotation around a centre which centre can be a centre point i.e. in one planar or a centre axis i.e. having a longitudinal extension.

"Axial movement" means any movement in an axial direction. Such movement can be a strictly translational movement or include a rotational movement which thus makes it a "Helically movement" as this is meant to be the combination of an axial movement and a rotational movement.

"Telescopic" is meant to cover the situation in which a movable element moves out from, and/or into, a base element. The telescopic movement can be either translational or include a rotation thus making the telescopic movement helical.

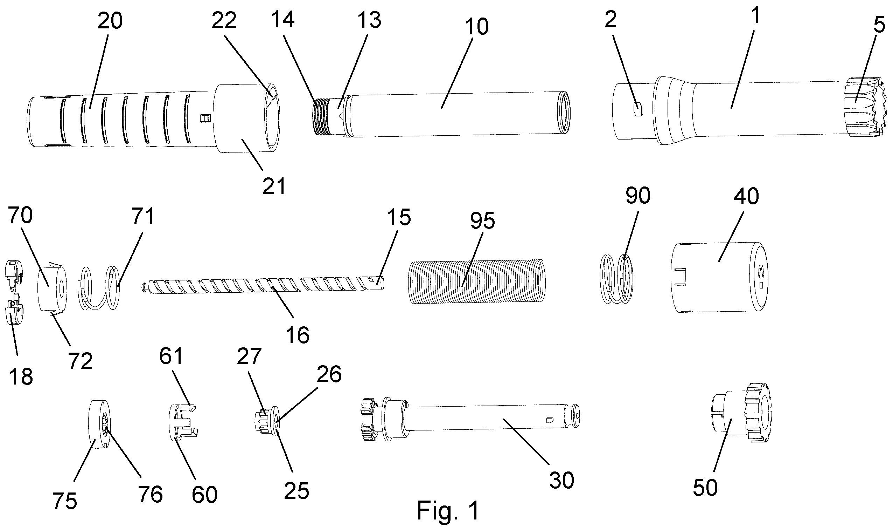

**Figure 1** disclose an exploded view of the injection device according to a first example of the invention and **figure 2A** shows the same in a cross-sectional view.

The injection device comprises a housing structure which is made up from a housing part 1 and a cartridge holder 20. The housing part 1 contains the dosing mechanism, which is shown in an enlarged version in **figure 2B****,** and the cartridge holder 20 contains the cartridge 10. The housing part 1 and the cartridge holder 20 is either permanently attached thus making up a pre-filled injection device or the cartridge holder 20 can be removed from the housing part 1 for replacement of the cartridge 10. This would hence be a durable injection device.

In the disclosed example, the injection device is a durable injection device and the cartridge holder 20 is proximally provided with a ring-shaped element 21 which makes up an integral part of the cartridge holder 20 and which in one example can be moulded integral with the remaining part of the cartridge holder 20. The inner surface of the cartridge holder 20 or rather the ring-shaped element 21 is provided with a track 22 which is able to engage with a guiding protrusion 2 provided on the housing part 1 thus making up a bayonet coupling. Both the track 22 and the guiding protrusion 2 are preferably provided in pairs. The user is henceforth able to uncouple the cartridge holder 20 from the housing part 1 when replacing the cartridge 10.

The cartridge 10 is in the disclosed example a standard glass cartridge which distally is sealed by a pierceable septum 11 and proximally closed by a movable plunger 12. In the disclosed example, the cartridge 10 is distally provided with an adapter top 13 which carries a thread 14 such that a non-shown pen needle can be attached to the thread 14. The adapter top 13 is preferably press fitted onto the cartridge 10 and inrotatable secured by the cartridge holder 2 when mounted as it is commonly known in the art. Alternatively, the thread 14 could be a provided on the housing structure of the injection device. The thread 14 could further be substituted by an alternative coupling means such as a bayonet coupling or a luer coupling for attaching the pen needle to the cartridge 10 or to the housing structure.

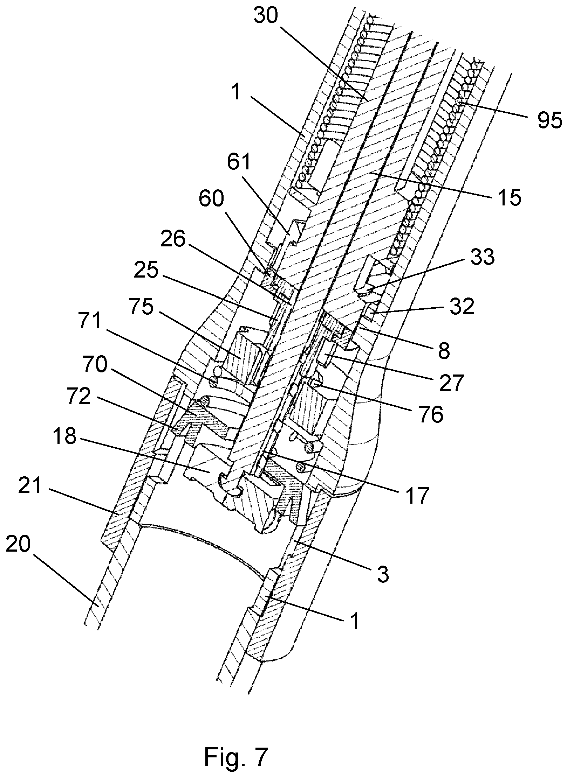

The cartridge 10, when mounted, is urged in the distal direction to abut the cartridge holder 20 by a distal compression spring 71 which distally abut a slider 70 and proximally abut against the housing structure. This slider 70 is provided with a pair of radial arms 72 which engage longitudinal tracks 3 (see e.g. figure 7) in the housing part 1 such that the slider 71 can only slide translational relative to the housing part 1 but are unable to rotate.

The drive mechanism inside the housing part 1 comprises a piston rod 15 which can be moved in the distal direction to thereby move the movable plunger 12 forward inside the cartridge 10. A piston rod foot 18 can be provided between the piston rod 15 and the movable plunger 12 to better distribute the force onto the movable plunger 12. In the disclosed example, the piston rod foot 18 is made from two parts which are radially clicked on to the distal end of the piston rod 15.

The piston rod 15 is provided with an outer thread 16 on an outer surface and a longitudinal track structure 17 (best seen in figure 7). The outer thread 16 on the piston rod 15 engages an inner thread 26 provided in a nut element 25 such that the piston rod 15 is moved helically when the piston rod 15 is rotated relatively to the nut element 25 as will be explained.

The longitudinal track structure 17 in the piston rod 15 is engaged by a dose tube 30 having internal engaging means engaging the longitudinal track struck structure 17 such that whenever the dose tube 30 is rotated the piston rod 15 rotates together with the dose tube 30. In one example the track structure 17 comprises flat surfaces which are engaged by the dose tube 30 in a key-like structure.

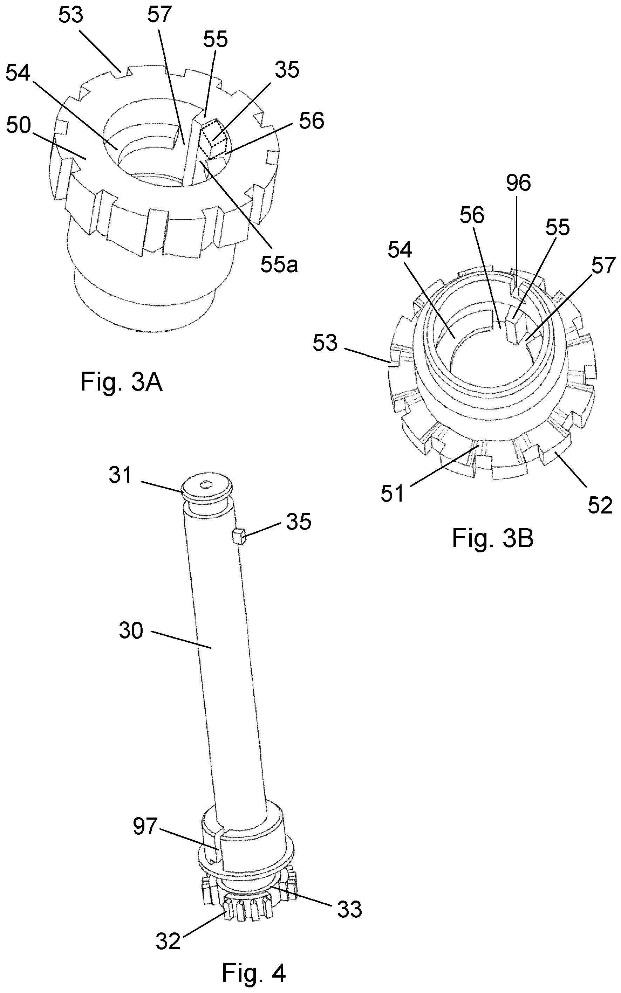

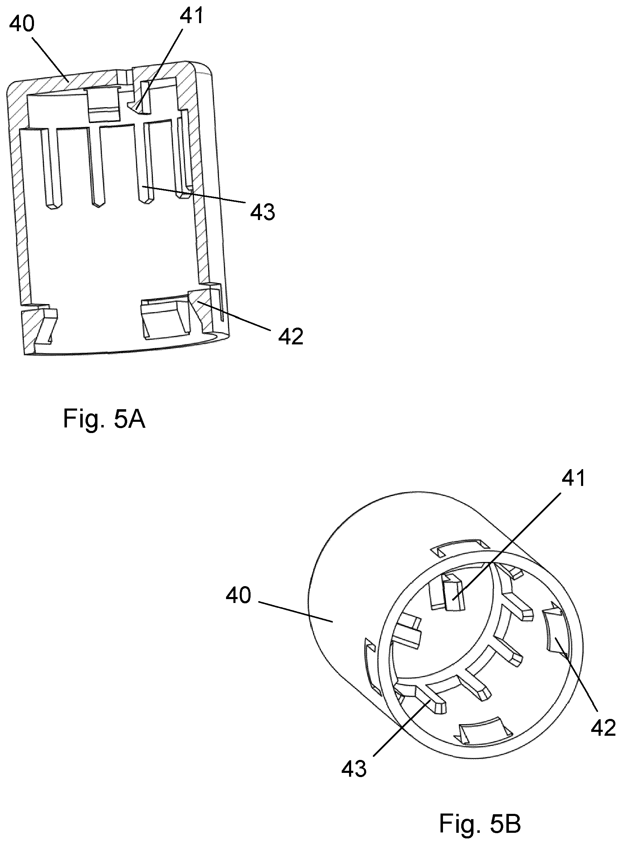

The dose tube 30 which is disclosed in **figure 4** is proximally provided with a circular ridge 31 which is engaged by the dose selector 40. The dose selector 40 disclosed in **figure 5A-B**is for that purpose provided with a number of proximal click-arms 41 on the inside which engages the circular ridge 31 on the dose tube 30 such that the dose selector 40 and the dose tube 30 move together axially but are able to rotate relatively to each other.

Distally, the dose selector 40 is provided with a plurality of distal resilient arms 42 which engages the housing part 1 such that the dose selector 40 can slide axially relatively to the housing part 1 but are prevented from being fully released from the housing structure. Preferably, these resilient arms 42 engages behind a plurality of outwardly pointing ribs 5 on the housing part 1, the use of which will be explained

The dose selector 40 is urged in the proximal direction by a compression spring 90 which is encompassed between the dose selector 40 and a connector element 50 and forces the distal resilient arms 42 to abut against the proximal surface of the outwardly pointing ribs 5 as best seen in figure 2B.

The connector element 50 which is disclosed in **figure 3A-B**connects to a torsion spring 95 which at its opposite distal end is coupled to the drive tube 30 such that a torque is build up in the torsion spring 95 when the connector element 50 and the drive tube 30 are rotated relatively to each other as will be explained.

Both the connector element 50 and the drive tube 40 are provided with L-shaped tracks 96, 97 for securing the torsion spring 95. The ends of the torsion spring 95 are for that purpose preferably provided with inwardly pointing hooks which can be guided translation through the longitudinal part of the L-shaped tracks 96, 97 and rotated into engagement with the respective part.

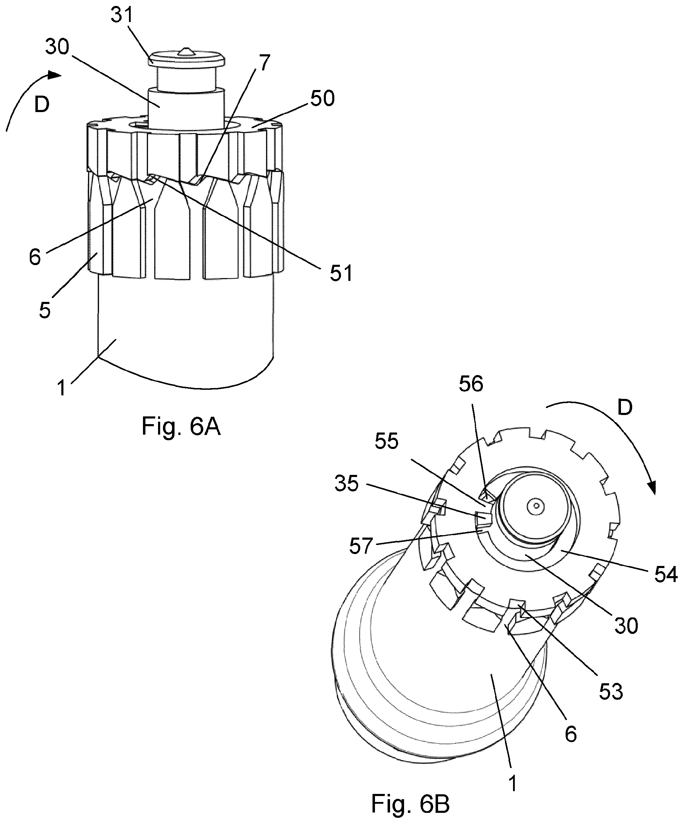

The engagement between the connector element 50 and housing part 1 is disclosed in **figure 6A-B****.** As seen, the housing part 1 is proximally provided with a number of outwardly pointing ribs 5 which are axially separated by guiding tracks 6 which are able to guide the dose selector 40 translational during injection.

At the proximal end of the housing part 1 a number of axially pointing proximal V-shaped ratchet teeth 7 are provided which engages a number of similar distal ratchet teeth 51 provided on the distal end surface of the connector element 50 as best seen in figure 3B. When the compression spring 90 urges the connector element 50 against the housing part 1, the ratchet interface (7, 51) between the axially pointing proximal V-shaped ratchet teeth 7 and the distal ratchet teeth 51 provided on the connector element 50 is able to hold the torque of torsion spring 95 as will be explained.

The drive mechanism is disclosed in **figure 7** and comprises the piston rod 15, the drive tube 30 and the nut member 20.

The drive tube 30 which is disclosed in further details in figure 4 is distally provided with a number of radial teeth 32 which between injections engages similar teeth 8 provided on the inner surface of the housing part 1. This engagement (32, 8) secures the drive tube 30 against rotation relatively to the housing part 1.

The nut member 25 which internally is provided with an inner thread 26 threaded to the outer thread 16 of the piston rod 15 is coupled to the drive tube 30 by a click element 60 such that the nut element 25 is axially movable together with the drive tube 30 but able to rotate relatively to the drive tube 30. The click element 60 is for that purpose provided with a number of proximally pointing click arms 61 which grabs behind a distal shelf 33 provided on the drive tube 30 as disclosed in figure 4 and in figure 7.

On the outer surface, the nut element 25 is provided with a number of splines 27 which are able to engage similar internal splines 76 provided in a housing ring 75 which is rotationally and axially secured in the housing part 1 and thus comprised in the housing structure. As best seen in figure 1 and figure 2B, the housing ring 75 is attached to the housing part 1 by a tongue and groove connection. In an alternative embodiment the housing ring 75 is moulded as an integral structure of the housing part 1.

Between injections the nut element 25 is not fixated and is able to rotate freely. As a consequence, the piston rod 15 which is threaded (16, 26) to the nut element 20 is free to move axially. Between injections, the drive tube 30 is secured against rotation by the engagement of the radial teeth 32 on the drive tube 30 with the internal teeth 8 in the housing part 1 and a translational movement (without rotation) of the piston rod 15 is possible. During such translation of the piston rod 15, the nut element 25 is forced to rotate and is also free to rotate. As a result, should the content of the cartridge 10 expand e.g. due to exposure to frost, the piston rod 15 can be moved proximally by the movable plunger 12 inside the cartridge 10.

During injection, the drive tube 30 is pushed distally out of engagement (32, 8) with the housing part 1 as will be explained. The nut member 25 which is axially coupled to the drive tube 30 follows the axial movement of the drive tube 30 and couples to the housing ring 75 via the splines (27, 76). When the drive tube 30 is moved out of its engagement with the housing part 1, the torsion spring 95 will rotate the drive tube 30. A rotation of the drive tube 30 and hence the piston rod 15 in this situation moves the piston rod 15 helical forward (= distally) due to the threaded engagement (16, 26) with the nut element 20 which is inrotatable secured by the engagement (27, 76) with the housing ring 75.

The connector element 50 shown in further details in figure 3A-B surrounds the drive tube 30 as disclosed in figure 6A and is on an outer surface provided with a plurality of outwardly pointing teeth 52 which are separated by rectangular openings 53.

These rectangular openings 53 are engaged by the inside splines 43 provided in the dose selector 40 disclosed in figure 5A-B such that a rotation of the dose selector 40 is transformed to a similar rotation of the connector element 50. The engagement between the rectangular openings 53 and the inside splines 43 are such that the dose selector 40 and the connector element 50 rotate together one-to-one in both rotational directions, however, the dose selector 40 is able to move translational in relation to the connector element 50.

The connector element 50 is further provided with an internal rib 54 and a longitudinal flange 55 as disclosed in figure 3A-B. The open area between the longitudinal flange 55 and the internal rib 54 defines two openings 56, 57. A first injection opening 56 and a second release opening 57, the use of which will be explained in the following. Both the injection opening 56 and the release opening 57 has a width such that a radial protrusion 35 provided proximally on the drive tube 30 can pass translational through these openings 56, 57.

In order to perform an injection, the user needs first to insert a cartridge 10 into the cartridge holder 20 and connect the cartridge holder 20 to the housing part using the bayonet coupling (22, 2). Once a cartridge 10 containing the liquid drug is inserted, the injection device is ready to be used.

When the injection device is ready for dose selection, the proximal compression spring 90 urges the dose selector 40 and the drive tube 30 in the proximal direction such that the radial teeth 32 on the drive tube 30 engages the internal teeth 8 inside the housing part 1 whereby the drive tube 30 is secured against rotation. This position is disclosed in figure 7.

The user then rotates the dose selector 40 and the connector element 50 in the counter clock-wise direction when viewed from a position distal to the injection device. This is indicated by the arrow "D" in figure 6A-B in which the dose mechanism is viewed from a position proximal to the injection device hence making the arrow point in the clock-wise direction.

Figure 6B disclose the position wherein the radial protrusion 35 is located above the release opening 57 and abutting the flange 55 at a first side (the initial position). During rotation ("D") of the connector element 50 away from this initial position, the rib 54 on the connector element 50 slides under the radial protrusion 35 on the drive tube 30 which prevents the user from moving the drive tube 30 in the distal direction. Since the torsion spring 95 is operational between the connector element 50 and the drive tube 30, a rotation of the connector element 50 while the drive tube 30 is locked to the housing part 1 strains the torsion spring 95 such that a torque is build up in the torsion spring 95.

The torque so build up in the torsion spring 95 is, as previously explained, secured by the engagement between the V-shaped ratchet teeth 7 on the housing part 1 and the distal ratchet teeth 51 provided distally on the connector element 50. The connector element 55 being urged against the housing part 1 by the compression spring 90 operational between the dose selector 50 and the connector element 50.

Once the rib 54, the flange 55 and the injection opening 56 has been rotated to a position in which the radial protrusion 35 on the drive tube 30 is aligned with the injection opening 56 (the dose position) it is possible to translate the drive tube 30 in the distal direction. In this injection position, the radial protrusion 35 preferably abut the flange 55 on the second opposite side 55a of the flange 55. The injection position or dose position is indicated in figure 3A in which the radial protrusion 35 is shown with broken lines.

When the injection opening 56 and the radial protrusion 35 is aligned the predetermined fixed dose has been selected. In order to expel the selected fixed dose, the user presses the dose dial 40 in the distal direction. During this translational movement of the dose dial 40, the inside splines 43 engages the axial guiding tracks 6 in the housing part 1 which secures both the dose selector 40 and the connector 50 from rotation relatively to the housing structure during dose expelling.

The distal movement of the dose selector 40 is transmitted to a similar translational movement of the drive tube 30 such that the radial teeth 32 disengages from the internal teeth 8 in the housing part 1. At the same time the splines 27 on the nut element 25 engages with the internal splines 76 in the housing ring 75 thereby locking the nut element 25 to the housing part 1.

When the radial teeth 32 disengages the internal teeth 8 in the housing part 1, the torque stored in torsion spring 95 rotates the drive tube 30. The torque stored in the torsion spring 95 is transmitted to a rotation of the drive tube 30 as the connector element 50 during dose expelling is secured to the housing part 1 by the inside splines 43 on the dose selector 40 simultaneously engaging both the rectangular openings 53 in the connector element 50 and the guiding tracks 6 in the housing part 1. Further, the connector element 50 is pressed firmly against the housing part 1 thus fully securing the interface between the proximal V-shaped ratchet teeth 7 and distal ratchet teeth 51 on the connector element 50.

When the drive tube 30 has been set free to be rotated by the torsion spring 95, the drive tube 30 will continue to rotate until the radial protrusion 35 again abut the flange 55. In this position, and when the user removes the finger from the dose selector 40, the protrusion 35 will be moved proximally up through the release opening 57 by the proximal compression spring 90 urging the drive tube 30 and the does selector 40 in the proximal direction. The end position is illustrated in figure 6B. In addition to being the end position of an injection it is also the start position for selecting a new fixed dose to be delivered hence also referred to as the initial position.

The rotation of the drive tube 30 for each dose selection is thus a little less than 360 degrees and the volume of the fixed dose is determined by the manufacture of the injection device via the selection of the pitch on the threaded engagement (16, 26) between the piston rod 15 and the nut element 25. This selection determines the distance the piston rod 15 is moved in the distal direction for each dose release.

Once the piston rod 15 and the plunger 12 has been moved to the distal end of the cartridge 10 and the content of the cartridge 10 has been expelled, the user must replace the cartridge 10 with a new full cartridge 10. This is done by first removing the cartridge holder 20 and discard the empty cartridge 10. Thereafter a new cartridge 10 must be inserted into the cartridge holder 20 and the piston rod 15 must be pushed back to its initial position.

When pushing the piston rod 15 in the proximal direction, the key engagement between the piston rod 15 and the drive tube 30 secures that the piston rod 15 moves translational without rotation. This is due to the toothed engagement (8, 32) between the drive tube 30 and the housing part 1 which prevents the drive tube 30 from rotation.

During the non-rotational translational movement of the piston rod 15 in the proximal direction, the nut element 25 is forced to rotate due to the threaded engagement (16, 26) between the outer thread 16 on the piston rod 15 with the inner thread 26 in the nut element 25.

Usually, a user pushing the piston rod 15 proximally with the use of the fingers would only push the piston rod 15 to a position in which the piston rod foot 18 is aligned with the housing part 1. When the cartridge holder 20 thereafter is attached to the housing part 1 via the bayonet coupling (2, 22) the helical engagement between the helical track 22 and the guiding protrusion 2 makes the plunger 12 inside the cartridge 10 push the piston rod 15 the last part of the way such that the piston rod foot 18 abut the plunger 12 in the start position of the new cartridge 10 thereby avoiding any air-gap and the also eliminating the subsequent need for performing an air-shot.

Some preferred embodiments have been shown in the foregoing, but it should be stressed that the invention is not limited to these, but may be embodied in other ways within the subject matter defined in the following claims.

## Claims

1. A torsion spring driven injection device for dispensing a number of predetermined and equally sized fixed dose volumes of a liquid drug, comprising:
A housing structure (1, 20) securing a cartridge (10) containing the liquid drug to be dispensed,
A piston rod (15) for driving the liquid drug out from the cartridge (10),
A rotatable drive structure (30) operational engaging the piston rod (15) such that the piston rod (15) is rotated together with the rotatable drive structure (30) at least during dosing,
A nut element (25) secured to the housing structure (1, 20) at least during dosing and threaded (16, 26) to the piston rod (15) such that piston rod (15) is moved helically upon rotation of the piston rod (15) relatively to the nut element (25),
A connector element (50) which is coupled to the housing structure (1, 2) by a ratchet interface (7, 51) allowing the connector element (50) to rotate relatively to the housing structure (1, 2),
A torsion spring (95) operable coupled between the rotatable drive structure (30) and the connector element (50), such that a torque is build up in the torsion spring (95) by rotation of the connector element (50) relatively to the rotatable drive structure (30) and wherein the ratchet interface (7, 51) between the housing structure (1, 20) and the connector element (50) is able to hold the connector element (50) in its rotational position against the torque of the torsion spring (95),
The rotatable drive structure (30) being axially movable between a first position and a second position;
the first position; being a position wherein the rotatable drive structure is non-rotationally secured to the housing structure (1, 20), and
the second position; being a position wherein the rotatable drive structure is released from the housing structure (1, 20) and able to rotate under influence of the torque stored in the torsion spring (95),
wherein the torsion spring (95) is strained when selecting one of the numbers of predetermined and equally sized fixed dose volumes by rotation of the connector element (50) relatively to the rotatable drive structure (30) with the rotatable drive structure (30) maintained in the first position; the connector element (50) being rotatable from an initial position to a dose position, and
wherein the selected one of the numbers of predetermined and equally sized fixed dose volumes is individually released when the rotational drive structure (30) is moved axially from the first position and to the second position allowing the rotatable drive structure (30) to rotate from the dose position to the initial position under influence of the torsion spring (95), wherein,
the rotatable drive structure (30) and the connector element (50) are provided with engaging means (35, 56) only allowing the rotatable drive structure (30) to move axially from the first position to the second position when the engaging means (35, 56) provided on the drive structure (30) and the connector element (50), respectively are aligned when the rotatable drive structure (30) has been rotated to, and is positioned, in the dose position.

2. A torsion spring driven injection device according to claim 1, wherein a rotational selection element (40) for rotational selecting one of the numbers of predetermined and equally sized fixed dose volumes to be ejected is provided at a proximal end of the housing structure (1, 20).

3. A torsion spring driven injection device according to claim 2, wherein the rotational selection element (40) is rotationally coupled to the connector element (50) such that the connector element (50) rotates together with the selection element (40) when selecting the predetermined and equally sized fixed dose volume and wherein the selection element (40) is axially movable relatively to the connector element (50), such that the selection element (40) is allowed to be moved axially in relation to the connector element (50).

4. A torsion spring driven injection device according to claim 2 or 3, wherein the rotational selection element (40) is axially coupled to the rotatable drive structure (30) such that the selection element (40) and the rotatable drive structure (30) move together in the axial direction to move the rotatable drive structure (30) from the first position to the second position to thereby release the rotatable drive structure (30) and wherein the selection element (40) and the rotatable drive structure (30) are allowed to rotate in relation to each other.

5. A torsion spring driven injection device according to claim 2, 3 or 4, wherein a compression spring (90) urges the selection element (40) and the rotatable drive structure (30) in the proximal direction.

6. A torsion spring driven injection device according to anyone of the claims 2 to 5, wherein the rotational dose selection element (40) is guided axially by guiding means (5, 6) in the housing structure (1, 2).

7. A torsion spring driven injection device according to any of the preceding claims, wherein the rotatable drive structure (30) comprises a longitudinal drive tube (30) provided with a radial protrusion (35) as one of the engagement means (35, 56).

8. A torsion spring driven injection device according to claim 7, wherein the connector element (50) is provided with a first axial opening (56) as one of the engagement means (35, 56).

9. A torsion spring driven injection device according to claim 8, wherein the radial protrusion (35) provided on the drive tube (30) is able to pass axially through the first axial opening (56) in the connector element (50) when the first axial opening (56) and the radial protrusion (35) are aligned to thereby initiate an ejection.

10. A torsion spring driven injection device according to claim 9, wherein the connector element (50) is provided with a second axial opening (57).

11. A torsion spring driven injection device according to claim 10, wherein the radial protrusion (35) provided on the rotatable drive tube (30) is able to pass axially through the second axial opening (57) in the connector element (50) when the second axial opening (57) and the radial protrusion (35) are aligned at the end of an ejection.

12. A torsion spring driven injection device according to any of the claims 7 to 11, wherein the piston rod (15) is provided with an outer thread (16) and a longitudinal guiding surface (17) such a groove.

13. A torsion spring driven injection device according to claim 12, wherein the housing structure internally carries a nut element (25) having an inner thread (26) engaging the outer thread (16) on the piston rod (15) and wherein the rotatable drive tube (30) is provided with internal engaging means engaging the longitudinal guiding surface (17) on the piston rod (15) such that the piston rod (15) is rotatable together with the rotatable drive tube (30) and moved helically when the drive tube (30) and the piston rod (15) are rotated relatively to the nut element (25).

14. A torsion spring driven injection device according to claim 13, wherein the nut element (25) is axially movable and coupled to the rotatable drive tube (30) to move axially together with the drive tube (30).

15. A torsion spring driven injection device according to claim 14, wherein the nut element (25) is rotational released from the housing structure in the first position of the rotatable drive tube (30) and rotationally secured to the housing structure in the second position of the rotatable drive tube (30).

## Patentansprüche

1. Torsionsfedergetriebene Injektionsvorrichtung zur Abgabe einer Anzahl von vorbestimmten und gleich großen festen Dosismengen eines flüssigen Arzneimittels, umfassend:
eine Gehäusestruktur (1, 20), die eine Patrone (10) sichert, die das abzugebende flüssige Arzneimittel enthält,
eine Kolbenstange (15) zum Heraustreiben des flüssigen Arzneimittels aus der Patrone (10),
eine drehbare Antriebsstruktur (30), die mit der Kolbenstange (15) in Wirkeingriff ist, sodass die Kolbenstange (15) zusammen mit der drehbaren Antriebsstruktur (30) zumindest während der Dosierung gedreht wird,
ein Mutterelement (25), das an der Gehäusestruktur (1, 20) zumindest während der Dosierung befestigt und mit der Kolbenstange (15) verschraubt (16, 26) ist, sodass die Kolbenstange (15) bei Drehung der Kolbenstange (15) in Bezug auf das Mutterelement (25) schraubenförmig bewegt wird,
ein Verbindungselement (50), das mit der Gehäusestruktur (1, 2) durch eine Klinkenschnittstelle (7, 51) gekoppelt ist, die eine Drehung des Verbindungselements (50) in Bezug auf die Gehäusestruktur (1, 2) ermöglicht,
eine Torsionsfeder (95), die zwischen der drehbaren Antriebsstruktur (30) und dem Verbindungselement (50) wirkverbunden ist, sodass ein Drehmoment in der Torsionsfeder (95) durch Drehung des Verbindungselements (50) in Bezug auf die drehbare Antriebsstruktur (30) aufgebaut wird, und wobei die Klinkenschnittstelle (7, 51) zwischen der Gehäusestruktur (1, 20) und dem Verbindungselement (50) in der Lage ist, das Verbindungselement (50) in seiner Drehposition gegen das Drehmoment der Torsionsfeder (95) zu halten,
wobei die drehbare Antriebsstruktur (30) axial zwischen einer ersten Position und einer zweiten Position beweglich ist;
wobei die erste Position eine Position ist, in der die drehbare Antriebsstruktur drehfest an der Gehäusestruktur (1, 20) gesichert ist, und
wobei die zweite Position eine Position ist, in der die drehbare Antriebsstruktur von der Gehäusestruktur (1, 20) gelöst ist und sich unter Einfluss des in der Torsionsfeder (95) gespeicherten Drehmoments drehen kann,
wobei die Torsionsfeder (95) gespannt wird, wenn eine der Anzahl von vorbestimmten und gleich großen festen Dosismengen durch Drehung des Verbindungselements (50) in Bezug auf die drehbare Antriebsstruktur (30) ausgewählt wird, wobei die drehbare Antriebsstruktur (30) in der ersten Position gehalten wird; wobei das Verbindungselement (50) aus einer anfänglichen Position in eine Dosisposition drehbar ist, und
wobei die ausgewählte der Anzahl von vorbestimmten und gleich großen festen Dosismengen einzeln freigegeben wird, wenn die drehbare Antriebsstruktur (30) axial aus der ersten Position in die zweite Position bewegt wird, wodurch der drehbaren Antriebsstruktur (30) ermöglicht wird, sich unter Einfluss der Torsionsfeder (95) aus der Dosisposition in die anfängliche Position zu drehen, wobei
die drehbare Antriebsstruktur (30) und das Verbindungselement (50) mit Eingriffsmitteln (35, 56) versehen sind, die es der drehbaren Antriebsstruktur (30) nur dann ermöglichen, sich axial aus der ersten Position in die zweite Position zu bewegen, wenn die Eingriffsmittel (35, 56), die an der Antriebsstruktur (30) und dem Verbindungselement (50) vorgesehen sind, jeweils ausgerichtet sind, wenn die drehbare Antriebsstruktur (30) in die Dosisposition gedreht wurde und in dieser positioniert ist.

2. Torsionsfedergetriebene Injektionsvorrichtung nach Anspruch 1, wobei ein Drehauswahlelement (40) zur Drehauswahl einer der Anzahl von vorbestimmten und gleich großen festen Dosismengen, die ausgestoßen werden sollen, an einem proximalen Ende der Gehäusestruktur (1, 20) vorgesehen ist.

3. Torsionsfedergetriebene Injektionsvorrichtung nach Anspruch 2, wobei das Drehauswahlelement (40) drehbar mit dem Verbindungselement (50) gekoppelt ist, sodass sich das Verbindungselement (50) zusammen mit dem Auswahlelement (40) dreht, wenn die vorbestimmte und gleich große feste Dosismenge ausgewählt wird, und wobei das Auswahlelement (40) in Bezug auf das Verbindungselement (50) axial bewegbar ist, sodass das Auswahlelement (40) in Bezug auf das Verbindungselement (50) axial bewegt werden kann.

4. Torsionsfedergetriebene Injektionsvorrichtung nach Anspruch 2 oder 3, wobei das Drehauswahlelement (40) axial mit der drehbaren Antriebsstruktur (30) gekoppelt ist, sodass sich das Auswahlelement (40) und die drehbare Antriebsstruktur (30) gemeinsam in der axialen Richtung bewegen, um die drehbare Antriebsstruktur (30) aus der ersten Position in die zweite Position zu bewegen, um dadurch die drehbare Antriebsstruktur (30) freizugeben, und wobei sich das Auswahlelement (40) und die drehbare Antriebsstruktur (30) in Bezug zueinander drehen können.

5. Torsionsfedergetriebene Injektionsvorrichtung nach Anspruch 2, 3 oder 4, wobei eine Druckfeder (90) das Auswahlelement (40) und die drehbare Antriebsstruktur (30) in die proximale Richtung drängt.

6. Torsionsfedergetriebene Injektionsvorrichtung nach einem der Ansprüche 2 bis 5, wobei das Drehdosisauswahlelement (40) durch Führungsmittel (5, 6) in der Gehäusestruktur (1, 2) axial geführt ist.

7. Torsionsfedergetriebene Injektionsvorrichtung nach einem der vorhergehenden Ansprüche, wobei die drehbare Antriebsstruktur (30) ein Längsantriebsrohr (30) umfasst, das mit einem radialen Vorsprung (35) als eines der Eingriffsmittel (35, 56) versehen ist.

8. Torsionsfedergetriebene Injektionsvorrichtung nach Anspruch 7, wobei das Verbindungselement (50) mit einer ersten axialen Öffnung (56) als eines der Eingriffsmittel (35, 56) versehen ist.

9. Torsionsfedergetriebene Injektionsvorrichtung nach Anspruch 8, wobei der radiale Vorsprung (35), der an dem Antriebsrohr (30) vorgesehen ist, in der Lage ist, axial durch die erste axiale Öffnung (56) in dem Verbindungselement (50) hindurchzutreten, wenn die erste axiale Öffnung (56) und der radiale Vorsprung (35) ausgerichtet sind, um dadurch ein Ausstoßen auszulösen.

10. Torsionsfedergetriebene Injektionsvorrichtung nach Anspruch 9, wobei das Verbindungselement (50) mit einer zweiten axialen Öffnung (57) versehen ist.

11. Torsionsfedergetriebene Injektionsvorrichtung nach Anspruch 10, wobei der radiale Vorsprung (35), der an dem Antriebsrohr (30) vorgesehen ist, in der Lage ist, axial durch die zweite axiale Öffnung (57) in dem Verbindungselement (50) hindurchzutreten, wenn die zweite axiale Öffnung (57) und der radiale Vorsprung (35) am Ende eines Ausstoßens ausgerichtet sind.

12. Torsionsfedergetriebene Injektionsvorrichtung nach einem der Ansprüche 7 bis 11, wobei die Kolbenstange (15) mit einem Außengewinde (16) und einer Längsführungsfläche (17), wie einer Nut, versehen ist.

13. Torsionsfedergetriebene Injektionsvorrichtung nach Anspruch 12, wobei die Gehäusestruktur innen ein Mutterelement (25) mit einem Innengewinde (26) trägt, das mit dem Außengewinde (16) an der Kolbenstange (15) in Eingriff ist, und wobei das drehbare Antriebsrohr (30) mit inneren Eingriffsmitteln versehen ist, die mit der Längsführungsfläche (17) an der Kolbenstange (15) in Eingriff sind, sodass die Kolbenstange (15) zusammen mit dem drehbaren Antriebsrohr (30) drehbar ist und schraubenförmig bewegt wird, wenn das Antriebsrohr (30) und die Kolbenstange (15) in Bezug auf das Mutterelement (25) gedreht werden.

14. Torsionsfedergetriebene Injektionsvorrichtung nach Anspruch 13, wobei das Mutterelement (25) axial beweglich und mit dem drehbaren Antriebsrohr (30) gekoppelt ist, um sich zusammen mit dem Antriebsrohr (30) axial zu bewegen.

15. Torsionsfedergetriebene Injektionsvorrichtung nach Anspruch 14, wobei das Mutterelement (25) in der ersten Position des drehbaren Antriebsrohrs (30) drehend von der Gehäusestruktur gelöst ist und in der zweiten Position des drehbaren Antriebsrohrs (30) drehfest an der Gehäusestruktur gesichert ist.

## Revendications

1. Dispositif d'injection entraîné par ressort de torsion pour distribuer un certain nombre de volumes de doses fixes prédéterminés et de taille égale d'un médicament liquide, comprenant :
Une structure de logement (1, 20) fixant une cartouche (10) contenant le médicament liquide à distribuer,
Une tige de piston (15) pour expulser le médicament liquide hors de la cartouche (10),
Une structure d'entraînement rotative (30) étant opérationnellement en prise avec la tige de piston (15) de sorte que la tige de piston (15) est mise en rotation avec la structure d'entraînement rotative (30) au moins pendant le dosage,
Un élément d'écrou (25) fixé à la structure de logement (1, 20) au moins pendant le dosage et fileté (16, 26) avec la tige de piston (15) de telle sorte que la tige de piston (15) est déplacée de manière hélicoïdale lors de la rotation de la tige de piston (15) par rapport à l'élément d'écrou (25),
Un élément connecteur (50) est couplé à la structure de logement (1, 2) par une interface à cliquet (7, 51) permettant à l'élément connecteur (50) de tourner par rapport à la structure de logement (1, 2).
Un ressort de torsion (95) couplé de manière opérable entre la structure d'entraînement rotative (30) et l'élément connecteur (50), de telle sorte qu'un couple s'accumule dans le ressort de torsion (95) par rotation de l'élément connecteur (50) par rapport à la structure d'entraînement rotative (30) et dans lequel l'interface à cliquet (7, 51) entre la structure de logement (1, 20) et l'élément connecteur (50) est capable de maintenir l'élément connecteur (50) dans sa position de rotation à l'encontre du couple du ressort de torsion (95),
La structure d'entraînement rotative (30) étant axialement mobile entre une première position et une deuxième position ;
la première position ; étant une position dans laquelle la structure d'entraînement rotative est fixée de manière non rotative à la structure de logement (1, 20), et
la deuxième position ; étant une position dans laquelle la structure d'entraînement rotative est libérée de la structure de logement (1, 20) et capable de tourner sous l'influence du couple stocké dans le ressort de torsion (95),
dans lequel le ressort de torsion (95) est contraint lors de la sélection de l'un des nombres de volumes de doses fixes prédéterminés et de taille égale par rotation de l'élément connecteur (50) par rapport à la structure d'entraînement rotative (30) avec la structure d'entraînement rotative (30) maintenue dans la première position ; l'élément connecteur (50) étant rotatif d'une position initiale à une position de dose, et
dans lequel l'un des volumes de doses fixes prédéterminés et de taille égale sélectionné est libéré individuellement lorsque la structure d'entraînement rotative (30) est déplacée axialement de la première position à la deuxième position permettant à la structure d'entraînement rotative (30) de tourner de la position de dose à la position initiale sous l'influence du ressort de torsion (95), dans lequel,
la structure d'entraînement rotative (30) et l'élément connecteur (50) sont munis de moyens d'engagement (35, 56) permettant uniquement à la structure d'entraînement rotative (30) de se déplacer axialement de la première position à la deuxième position lorsque les moyens d'engagement (35, 56) prévus sur la structure d'entraînement (30) et l'élément connecteur (50) sont respectivement alignés lorsque la structure d'entraînement rotative (30) a été tournée vers, et est positionnée, dans la position de dose.

2. Dispositif d'injection actionné par ressort de torsion selon la revendication 1, dans lequel un élément de sélection de rotation (40) pour sélectionner par rotation l'un des nombres de volumes de doses fixes prédéterminés et de taille égale à éjecter est prévu à une extrémité proximale de la structure de logement (1, 20).

3. Dispositif d'injection commandé par ressort de torsion selon la revendication 2, dans lequel l'élément de sélection rotatif (40) est couplé en rotation à l'élément connecteur (50) de telle sorte que l'élément connecteur (50) tourne avec l'élément de sélection (40) lors de la sélection du volume de dose fixe prédéterminé et de taille égale et dans lequel l'élément de sélection (40) est mobile axialement par rapport à l'élément connecteur (50), de telle sorte que l'élément de sélection (40) peut être déplacé axialement par rapport à l'élément connecteur (50).

4. Dispositif d'injection actionné par ressort de torsion selon la revendication 2 ou 3, dans lequel l'élément de sélection rotatif (40) est couplé axialement à la structure d'entraînement rotative (30) de telle sorte que l'élément de sélection (40) et la structure d'entraînement rotative (30) se déplacent ensemble dans la direction axiale pour déplacer la structure d'entraînement rotative (30) de la première position à la deuxième position pour libérer ainsi la structure d'entraînement rotative (30) et dans lequel l'élément de sélection (40) et la structure d'entraînement rotative (30) sont autorisés à tourner l'un par rapport à l'autre.

5. Dispositif d'injection actionné par ressort de torsion selon la revendication 2, 3 ou 4, dans lequel un ressort de compression (90) pousse l'élément de sélection (40) et la structure d'entraînement rotative (30) dans la direction proximale.

6. Dispositif d'injection actionné par ressort de torsion selon l'une quelconque des revendications 2 à 5, dans lequel l'élément rotatif de sélection de dose (40) est guidé axialement par des moyens de guidage (5, 6) dans la structure de logement (1, 2).

7. Dispositif d'injection actionné par ressort de torsion selon l'une quelconque des revendications précédentes, dans lequel la structure d'entraînement rotative (30) comprend un tube d'entraînement longitudinal (30) pourvu d'une saillie radiale (35) en tant que l'un des moyens d'engagement (35, 56).

8. Dispositif d'injection actionné par ressort de torsion selon la revendication 7, dans lequel l'élément connecteur (50) est pourvu d'une première ouverture axiale (56) en tant que l'un des moyens d'engagement (35, 56).

9. Dispositif d'injection actionné par ressort de torsion selon la revendication 8, dans lequel la saillie radiale (35) prévue sur le tube d'entraînement (30) est capable de passer axialement à travers la première ouverture axiale (56) dans l'élément connecteur (50) lorsque la première ouverture axiale (56) et la saillie radiale (35) sont alignées pour initier une éjection.

10. Dispositif d'injection actionné par ressort de torsion selon la revendication 9, dans lequel l'élément connecteur (50) est muni d'une deuxième ouverture axiale (57).

11. Dispositif d'injection actionné par ressort de torsion selon la revendication 10, dans lequel la saillie radiale (35) prévue sur le tube d'entraînement rotatif (30) est capable de passer axialement à travers la deuxième ouverture axiale (57) dans l'élément connecteur (50) lorsque la deuxième ouverture axiale (57) et la saillie radiale (35) sont alignées à la fin d'une éjection.

12. Dispositif d'injection actionné par ressort de torsion selon l'une quelconque des revendications 7 à 11, dans lequel la tige de piston (15) est pourvue d'un filetage extérieur (16) et d'une surface de guidage longitudinale (17) telle qu'une rainure.

13. Dispositif d'injection actionné par ressort de torsion selon la revendication 12, dans lequel la structure de logement porte intérieurement un élément d'écrou (25) ayant un filetage interne (26) engageant le filetage extérieur (16) sur la tige de piston (15) et dans lequel le tube d'entraînement rotatif (30) est muni de moyens d'engagement internes engageant la surface de guidage longitudinale (17) de la tige de piston (15) de telle sorte que la tige de piston (15) tourne avec le tube d'entraînement rotatif (30) et est déplacée de manière hélicoïdale lorsque le tube d'entraînement (30) et la tige de piston (15) tournent par rapport à l'élément d'écrou (25).

14. Dispositif d'injection actionné par ressort de torsion selon la revendication 13, dans lequel l'élément d'écrou (25) est mobile axialement et couplé au tube d'entraînement rotatif (30) pour se déplacer axialement avec le tube d'entraînement (30).

15. Dispositif d'injection actionné par ressort de torsion selon la revendication 14, dans lequel l'élément d'écrou (25) est libéré en rotation de la structure de logement dans la première position du tube d'entraînement rotatif (30) et fixé de manière non rotative à la structure de logement dans la deuxième position du tube d'entraînement rotatif (30).
